# EUROPEAN PATENT APPLICATION

(11) **EP 3 443 948 A1**
(43) Date of publication of application: **20.02.2019**
(21) Application number: 17020359.0
(22) Date of filing: 17.08.2017
(51) Int. Cl.: A61K 8/02, A61Q 19/08

(54) **PARTICLE-CONTAINING COMPOSITION AND USES THEREOF**

(71) Applicant: The Boots Company PLC, Nottingham NG90 1BS (GB)
(72) Inventor: The designation of the inventor has not yet been filed
(74) Representative: Agashi, Kapil

(57) **Abstract**

The disclosed technology relates to a composition comprising particles and platelets, wherein the particles and platelets are present at a concentration (w/w) ratio of between about 30:70 and about 70:30. The disclosed technology further relates to the use and method of the disclosed technology in cosmetic applications.

## Description

### TECHNICAL FIELD

The disclosed technology relates to a composition comprising particles and platelets, wherein the concentration (w/w) ratio of particles to platelets is between about 30:70 and about 70:30. The disclosed technology further relates to the use and method of the disclosed technology in cosmetic applications.

### BACKGROUND OF THE INVENTION

Ageing is a multifactorial phenomenon. The ageing phenomenon may be due to one or more of genetic predisposition (known as chronological or intrinsic ageing) and one's physiological reaction to environmental stresses (sometimes referred to as actinic or extrinsic ageing). Actinic ageing appears skin specific and is defined as the effect of the external environment on the skin's biological response. The skin response to actinic ageing, which may be caused by sun and pollution exposure, as well as smoking, is typically associated with a lack of normal hydration, apparition of telangiectasia (spider veins), sagging of the skin, and/or reduced firmness of the skin. With sagging or reduced firmness the appearance of fine lines and wrinkles occurs. The sagging or reduced skin firmness may be explained by the fact that the elastic fibres of the dermal extracellular matrix, forming the support and conferring elasticity and strength to the skin are destroyed and become rare with age.

In addition, it is known that although skin covers the whole body, its thickness varies depending on the amount of wear and tear that body parts experience. On the face, neck, and décolleté skin differs from the rest of the body because skin is thinner than on most parts of the body. However, these three skin regions of the body frequently experience most exposure to environmental stresses, and other signs of ageing. As a result signs of aging of skin on face, neck and décolleté age are obvious to see, and because of biological difference between different skin regions of the body they age at different rates.

Increasingly, consumers wish to use cosmetic formulations that provide an immediate anti-ageing effect. In this regard, particles such as nylon powder are used in anti-ageing compositions of the art to disguise fine lines and provide a soft-focus effect that in turn provides a smoother-looking, even skin tone. However, if the mattifying effect is too high then a composition comprising such particles can make the skin look flat and dull. By contrast, some compositions use platelets such as talc and mica that can provide radiance, but if this effect is too high then the platelets can lead to an unnatural, shiny look and can make fine lines stand out.

Some compositions on the market combine particles and platelets. However, despite using a combination, these compositions using both particles and platelets fail to fully deal with the problems discussed above.

### SUMMARY OF THE INVENTION

It would be advantageous to have a composition that may be applied topically to provide a smoother, more youthful look without either looking flat or looking shiny. In one embodiment the composition may also be easier to apply topically as the composition may be more spreadable and feel more comfortable on the skin.

As used herein, the transitional term "comprising," which is synonymous with "including," "containing," or "characterized by," is inclusive or open-ended and does not exclude additional, un-recited elements or method steps. However, in each recitation of "comprising" herein, it is intended that the term also encompass, as alternative embodiments, the phrases "consisting essentially of" and "consisting of," where "consisting of" excludes any element or step not specified and "consisting essentially of" permits the inclusion of additional un-recited elements or steps that do not materially affect the basic, essential and novel characteristics of the composition, method or use under consideration.

In the present application, the term "about" may encompass ±10%, such as ±5%, for example ±2%, preferably ±1%.

As used herein reference to gel is used in the ordinary sense defined by IUPAC and is intended to include a non-fluid colloidal network or polymer network that is expanded throughout its whole volume by a fluid. The fluid may for instance be water or alcohol.

Unless otherwise indicated concentrations of each ingredient of the composition disclosed herein is on a weight (abbreviated to "w" or "wt") basis relative to the total composition disclosed herein.

Unless otherwise indicated various ingredients disclosed herein may be individual compounds, or in a mixture of compounds.

As used herein reference to skin is intended to include skin that covers the surface of the epidermis or stratum corneum, for example facial, or body or lip skin.

As used herein reference to eyelashes is intended to include hair that grows at the edge of the eyelid. Compositions intended for application to the eyelashes include mascaras.

As used herein reference to eyebrows is intended to include hair that grows above the eye that follows the shape of the lower margin of the brow ridges. Compositions intended for application to the eyebrows include mascaras and eyebrow gloss.

In the first aspect the present invention provides a composition comprising particles and platelets, wherein the concentration (w/w) ratio of particles to platelets is between about 30:70 and about 70:30. The inventors have surprisingly found that, through providing the correct level of balance between particles and platelets, an optimum, more youthful look is achieved, as determined in user trials.

In one embodiment the composition is a cosmetic composition. In a preferred embodiment, the composition is a colour cosmetic. In a preferred embodiment, the composition is a pigmented emulsion, more specifically a foundation.

In one embodiment the concentration (w/w) ratio of particles to platelets is between about 40:60 and about 60:40, preferably between about 45:55 and 55:45, more preferably about 50:50. In one embodiment the particles and/or the platelets are present at a concentration of between about 1 wt % and about 5 wt %, preferably between about 2 wt % and about 4 wt %, more preferably between about 2.5 wt % and about 3.5 wt %, more preferably about 3 wt %. In one embodiment the particles are polymer particles, preferably nylon particles. In one embodiment the platelets are derived from a mineral, preferably talc platelets or mica platelets.

In one embodiment the composition further comprises a silicone elastomer. In one embodiment the silicone elastomer is present at a concentration of between about 0.5 wt % and about 10 wt %, preferably between about 1 wt % and about 8 wt %, more preferably between about 2 wt % and about 6 wt %, more preferably between about 3 wt % and about 5 wt %, more preferably about 4 wt %. In one embodiment the silicone elastomer is a non-emulsifying elastomer, preferably a dimethicone crosspolymer.

In one embodiment the composition further comprises a film-forming polymer. In one embodiment the film-forming polymer is present at a concentration of between about 0.1 wt % and about 10 wt %, preferably between about 0.5 wt % and about 6 wt %, more preferably between about 1 wt % and about 3 wt %, more preferably about 2 wt %. In one embodiment the film-forming polymer comprises silicon. In one preferred embodiment, the film-forming polymer is a siloxysilicate, preferably trimethylsiloxysilicate.

A further aspect of the present invention provides a method of imparting a benefit to a composition of at least one of easy application, flawless finish upon application, evened out skin tone upon application, a less cakey look upon application, a more youthful look upon application, less noticeable pores after application and less noticeable fine lines after application, comprising application of the composition defined herein topically to the skin.

A further aspect of the present invention provides a cosmetic use of the composition defined herein. In one embodiment the cosmetic use imparts a benefit of at least one of easy application, flawless finish upon application, evened out skin tone upon application, a less cakey look upon application, a more youthful look upon application, less noticeable pores after application and less noticeable fine lines after application.

### DETAILED DESCRIPTION OF THE INVENTION

The disclosed technology provides a composition, methods and uses as disclosed above.

### Cosmetic

Compositions of the present invention may be applicable to any form of cosmetic that can provide anti-ageing benefits. "Anti-ageing", in a cosmetic sense, relates to cosmetics that visibly reduce the signs of ageing, and include cosmetics that improve the health of the skin.

Preferably the compositions of the present invention are cosmetic compositions, more preferably skincare cosmetic compositions. These cosmetic compositions include colour cosmetics, creams such as hand creams, face creams, day creams and night creams, moisturisers, cleansers, lotions such as face lotions, under-eye lotions and hand lotions, face washes, gels, cleansing wipes, washes such as body washes and facial washes, balms, such as shaving balms, peels, serums, oils and sunscreens.

In a preferred embodiment, the colour cosmetic of the present invention is a pigmented emulsion. Colour cosmetics of the present invention include foundations, concealers such as under-eye concealers, eye filler treatments, volume filler treatments, BB creams, CC creams, tinted moisturisers, eye brow gloss and primers. Preferably the colour cosmetic is a foundation.

The BB cream and CC cream are believed to be a tinted moisturiser containing sun protection. BB creams and CC creams, as understood by a person skilled in the art, typically comprise a primer, serum, moisturiser, foundation and sun protection, with a greater focus on skin care (for example a higher level of sun protection) in the CC cream.

In one embodiment the disclosed technology relates to the cosmetic use of a composition of the present invention (for example a pigmented emulsion).

### Particles and Platelets

The use of particles (otherwise known as spheres) and platelets (also known as flakes) in anti-ageing compositions is known in the art. Both particles and platelets are solid when stored in a container and upon application, and so typically are solid at ambient room temperature. These solids can be derived from any cosmetically suitable material. These solids can also be derived from more than one material, for example (1) some of the particles of the present invention could be derived from one material and some of the particles could be derived from a second, different material (in which case, for the purposes of calculating the concentration (w/w) ratio of particles to platelets, the concentration of all of the particles combined would be taken into account), or (2) each particle of the present invention could be derived from more than one material (such as the particle comprising a core of one material and a coating of a second, different material), or (3) a combination of (1) and (2).

The embodiments above are applicable to platelets as well as particles. For example (1) some of the platelets of the present invention could be derived from one material and some of the platelets could be derived from a second, different material (in which case, for the purposes of calculating the concentration (w/w) ratio of particles to platelets, the concentration of all of the platelets combined would be taken into account), or (2) each platelet of the present invention could be derived from more than one material (such as the platelet comprising a core of one material and a coating of a second, different material), or (3) a combination of (1) and (2).

In one embodiment, the particles are derived from a material that is different from the material of the platelets. By contrast, the particles are derived from a material that is the same as the material of the platelets (preferably the materials are different). Thus, the key difference in relation to the terms "particle" and "platelet" is not in relation to the material they are derived from but instead in relation to the shape of the solids. Both particles and platelets are present within the composition in a powder form, where the size of each particle or platelet is between 1 µm and 120 µm in diameter.

Minerals, such as talc or mica, as well as boron nitride, can be used to form platelets, particles, or both particles and platelets (preferably platelets). Synthetic equivalents to these minerals can also be used. For example fluorphlogopite, a synthetic material very similar to mica, may be used.

Polymers can be used to form particles, platelets or both particles and platelets (preferably particles). Polymers may be silicone or non-silicone based. Non-silicone based polymers include nylon, polyamides such as polyhexamethylene adipamide (PA66), polycaproamide (PA6), PA6.10, PA10.10 and PA12, polyesters, polyolefins, polymers based on a cellulose ester, such as cellulose acetate, cellulose propionate, rayon, viscose and polymers of the same family, acrylic polymers, such as poly(methyl methacrylate), and copolymers, copolymers in any proportions of these polymers, and blends between any of these polymers. Preferably the polymer is nylon, such as nylon 6/12, nylon 66, nylon 6, nylon 510 or nylon 1,6 (preferably nylon 6/12). Silica (or a combination of a polymer of silica) may also be used to form particles.

Examples of Sensient polymer particles include COVABEAD LH 85 (methyl methacrylate cross polymer with a matte effect), COVABEAD LH 170 (methyl methacrylate cross polymer with a creamy feeling), COVABEAD PMMA (polymethyl methacrylate with a powdery feeling), COVABEAD VELVET 10 (polymethyl methacrylate with a superior softness effect), COVABEAD VELVET 20 (polymethyl methacrylate with a superior ball-bearing effect) and COVABEAD PMMA 2 MUSI (polymethyl methacrylate with silica).

Suitable Shin Etsu silicone-based polymer particles include KMP-590 (polymethylsilsesquioxane at an average diameter of 2 µm), KMP-591 (polymethylsilsesquioxane at an average diameter of 5 µm), KSP-100 (vinyl dimethicone/ methicone silsesquioxane crosspolymer at an average diameter of 5 µm), KSP-101 (vinyl dimethicone/ methicone silsesquioxane crosspolymer at an average diameter of 12 µm), KSP-102 (vinyl dimethicone/ methicone silsesquioxane crosspolymer at an average diameter of 30 µm), KSP-105 (vinyl dimethicone/ methicone silsesquioxane crosspolymer at an average diameter of 2 µm), KSP-300 (diphenyl dimethicone/vinyl diphenyl dimethicone/silsesquioxane crosspolymer), KSP-411 (polysilicone-1 crosspolymer) and KSP-441 (polysilicone-22).

Borosilicates can be used to form particles, platelets or both particles and platelets. Suitable borosilicate platelets include violet interference pearl. Suitable borosilicate particles include HOLLOW CORE SILICATE R3178 from Sensient, a calcium aluminium borosilicate.

Glass can be used to form particles. Suitable glass particles include COVABEAD CRYSTAL from Sensient, which are transparent spherical beads.

The platelets and particles can be treated and many forms of treatments are familiar to the person skilled in the art. A common form of treatment is the coating of platelets and particles. The coating material may be a metal oxide, an ester, a fluoropolymer, a silicone, a hybridised complex, a wax, a lecithin, a chitosan, lauroyl lysine, alkoxysilane or a mixture thereof (in the form of a multi-layered system, for example). Specific examples of coating include triethoxycaprylylsilane, isopropyl titanium triisostearate, methicone, dimethicone, perfluorooctyl triethoxysilane, stearoyl glutamic acid, silica, carnauba wax, aloe, jojoba ester, hydrogenated lecithin, magnesium myristate, polyethylene wax and a combination of silane with polyhydroxystearic acid. Preferably the coating is a triethoxycaprylylsilane coating. Preferably the platelets are coated.

The solids may be porous (in other words the platelets and/or particles may comprise interstices through which liquid or air may pass) or may be non-porous. Similarly, the solids may have a smooth surface or a rough surface.

Particles are considered to be solids within a composition that have a high level of sphericity, and typically include spherical, rounded, angular, cubic shapes. By contrast, platelets have a low level of sphericity, and typically include cylindrical, acicular and flakey shapes.

An analysis of the aspect ratio of a solid can be used in order to determine whether a powder sample comprises particles or platelets or both. The aspect ratio of a three-dimensional solid is the length of the longest dimension compared to the shortest length of a dimension orthogonal to the first length. A particle in the context of the present invention is any solid that has an aspect ratio of less than 5. A platelet in the context of the present invention is any solid that has an aspect ratio of greater than 10. The skilled person would straightforwardly be able to determine the aspect ratio of a powder sample (and thus whether the sample comprises particles or platelets) through microscopic analysis (either using light microscopy or electron microscopy). Where a powder sample comprises both particles and platelets, the skilled person would also be able to determine the ratio of platelets and particles and the respective concentrations through microscopic analysis.

In the composition of the present invention, the particles and platelets are present at a concentration (weight/weight, w/w) of between about 30:70 and about 70:30. Preferably the particles and platelets are present at a concentration (w/w) of between about 40:60 and about 60:40. More preferably the particles and platelets are present at a concentration (w/w) of between about 45:55 and about 55:45. More preferably the particles and platelets are present at a concentration (w/w) of about 50:50.

In one embodiment of the present invention, the particles and/or the platelets are present at a concentration of between about 1 wt % and about 5 wt %. Preferably the particles and/or the platelets are present at a concentration of between about 2 wt % and about 4 wt %. More preferably the particles and/or the platelets are present at a concentration of between about 2.5 wt % and about 3.5 wt %. More preferably the particles and/or the platelets are present at a concentration of about 3 wt %.

### Silicone Elastomer

In one embodiment of the present invention, the composition further comprises a silicone elastomer. It has been found that, in the context of the present composition, the presence of a silicone elastomer surprisingly improves the ease of application, provides an elegant rheology and diffuses the light in a flattering manner (see the Example).

Preferably the silicone elastomer is a non-emulsifying elastomer. Non-emulsifying elastomers may include dimethicone crosspolymers. In one embodiment, the dimethicone is crosslinked with polysilicone, and in this regard Grant Industries provide such elastomers in a dimethicone carrier that has a viscosity of either 2 centiStokes (cSt) (GRANSIL DMG-2) or 3 cSt (GRANSIL DMG-3). In another embodiment, the dimethicone is crosslinked with vinyl dimethicone, and in this regard Shin Etsu provide such elastomers in a dimethicone carrier that has a viscosity of 6 cSt with a level of crosslinking that is either between 20% and 30% (KSG-16 and KSG-016F) or between 10% and 20% (KSG-19). However, it is most preferable that the dimethicone is crosslinked with dimethicone, and in this regard Dow Corning provide such elastomers in a dimethicone carrier that has a viscosity of either 2 cSt (EL-9240) or 5 cSt (EL-9241) (preferably the elastomer is EL-9240). Dimethicone crosspolymers are also supplied by General Electric (SFE 839).

Cross-linked organopolysiloxane elastomers useful in the present invention and processes for making them are further described in US Patents 4,970,252; 5,760,116; and 5,654,362. Commercially available elastomers may include a Dow Corning's 9040 silicone elastomer blend, or Shin Etsu's KSG-21.

The composition may include an emulsifying crosslinked polysilicone elastomer, such as a crosslinked organopolysiloxane elastomer, a non-emulsifying crosslinked polysilicone elastomer, such as a crosslinked organopolysiloxane elastomer, or a mixture thereof. The term "non-emulsifying," as used herein, defines crosslinked organopolysiloxane elastomers from which polyoxyalkylene units are absent. The elastomers may include dimethyl polysiloxanes crosslinked by Si-H sites on a molecularly spherical MQ resin. Emulsifying crosslinked organopolysiloxane elastomers include the crosslinked polymers described in US Patents 5,412,004; 5,837,793; and 5,811,487. The emulsifying elastomer comprised of dimethicone copolyol crosspolymer and dimethicone is commercially available from Shin Etsu under the trade name KSG-21. Crosslinked polysilicone elastomers known in the art include polysilicone-11, polysilicone-15 and polysilicone-2.

In one embodiment, the silicone elastomer is present at a concentration of between about 0.5 wt % and about 10 wt %. Preferably the silicone elastomer is present at a concentration of between about 1 wt % and about 8 wt %. More preferably the silicone elastomer is present at a concentration of between about 2 wt % and about 6 wt %. More preferably the silicone elastomer is present at a concentration of between about 3 wt % and about 5 wt %. More preferably the silicone elastomer is present at a concentration of about 4wt%.

### Film-Forming Polymer

In one embodiment of the present invention, the composition further comprises a filming-forming polymer. The film-forming polymer may comprise silicon or, alternatively, may not comprise silicon. Preferably the film-forming polymer comprises silicon.

In one embodiment the film-forming polymer comprising silicon is a MQ resin. Suitable MQ resins include trialkylsiloxysilicates such as a trimethoxysilicate available under the trade name SR1000, a mixture of cyclopentasiloxane and trimethylsiloxysilicate available under the trade name SS4230, a mixture of dimethicone and trimethylsiloxysilicate available under the trade name SS4267, a liquid trimethylsiloxysilicate available under the trade name SR399, a diisostearoyl trimethylolpropane siloxysilicate available under the trade name SF 1318, or a phenylpropylsiloxysilicate available under the trade name Silshine151, all of which are available from GE Silicones. MQ resins including trimethyoxysilicate are also provided by Dow Corning without carrier (MQ-1600 Solid Resin), with a cyclopentasiloxane carrier (RSN-0749 Resin) or with a dimethicone carrier (593 Fluid). Most preferably the film-forming polymer is RSN-0749 Resin.

In one embodiment the film-forming polymer comprising silicon may include a silicone acrylate copolymer, silicone methacrylate copolymer or a silicone gum or resin thereof, such as acrylates/polytrimethyl siloxymethacrylate (for example FA 4001 CM silicone acrylate, FA 4002 ID silicone acrylate, FA 4003 DM silicone acrylate, FA 4004 ID silicone acrylate and FA 4103 silicone acrylate emulsion, all available from Dow Corning) butyl acrylate/ hydroxypropyl dimethicone acrylate copolymer, isobutylmethacrylate/bis-hydroxypropyl dimethicone acrylate copolymer, acrylates/polytrimethylsiloxymethacrylate copolymer, dimethiconol, divinyldimethicone/ dimethicone copolymer, trimethylsiloxysilicate, polysilsesquioxane or trimethylsiloxysilicate/dimethiconol crosspolymer.

In one embodiment the film-forming polymers not comprising silicon may include synthetic gum, natural gum, synthetic polymer, natural polymer, polysaccharide thickening agent, associative thickener, anionic associative rheology modifier, nonionic associative rheology modifier, acrylates/C10-30 alkylacrylate crosspolymer, acrylates/aminoacrylates /C10-30 alkyl PEG- 20 itaconate copolymer, acrylates copolymer, acrylates/steareth-20 methacrylate copolymer, acrylates/beheneth-25 methacrylate copolymer, PEG-150/decyl alcohol/SMDI copolymer, PVP (polyvinylpyrrolidone homopolymer or copolymer, for example alkylated polyvinylpyrrolidone), PVM/MA decadiene crosspolymer, carbomer, PEG crosspolymer, acrylates/palmeth-25 acrylates copolymer, polysaccharide, polyacrylate, polyether-1, sodium magnesium silicate, sodium carbomer, sodium polyacrylate, sodium polymethacrylate, sodium polyacryloyldimethyl taurate, sodium acryloyldimethyl taurate copolymer, sodium carragenan, sodium carboxymethyl dextran, hydroxyethylcellulose, hydroxypropyl cyclodextran, bentonites, trihydroxystearin, aluminium-magnesium hydroxide stearate, xanthan gum, isoprene/MA/methoxy PEG-40 copolymer, VA/butyl maleate/isobornyl acrylate copolymer, dimethylacrylamide/acrylic acid/polystyrene/ethyl methacrylate copolymer, acrylates/octylacrylamide copolymer, polyvinyl alcohol, VP/ hexadecane copolymer, polybutene, polybutene, polyurethane, acrylate/octylcrylamide copolymer, adipic acid/diethylene glycol/glycerine crosspolymer, or trimethylpentanediol/ adipic acid/glycerin crosspolymer.

In one embodiment the film-forming polymer may include polyvinyl alcohol, VP/hexadecene copolymer, alkylated polyvinylpyrrolidone, acrylates copolymer, acrylates /actylacrylamide copolymer, polybutene, adipic acid/diethylene glycol/glycerin crosspolymer, trimethylpentanediol/adipic acid/glycerin crosspolymer, or polyurethane.

In one embodiment the film-forming polymer acts also as a silicone elastomer. However, it is more preferable, where the composition comprises both a silicone elastomer and a film-forming polymer, that the silicone elastomer is an ingredient that is different to the film-forming polymer.

In one embodiment the film-forming polymer is present at a concentration of between 0 wt % or about 0.1 wt % and about 10 wt %. Preferably the film-forming polymer is present at a concentration of between about 0.5 wt % and about 6 wt %. More preferably the film-forming polymer is present at a concentration of between about 1 wt % and about 3 wt %. More preferably the film-forming polymer is present at a concentration of about 2 wt %.

### Other Ingredients

The composition disclosed herein may optionally further comprise other ingredients. The other ingredients may include one or more of cosmetically acceptable pigments, antioxidants, skin conditioning agents, salicylic acid compounds, emulsifiers, surfactants preservatives, sequestering agents, chelating agents, vitamins, waxes, polar solvents, perfumes, pH adjusting agents, thickeners, viscosity modifying agents, gelling agents, diluents, carriers, sunscreen agents, propellants (such as dimethyl ether) and water soluble dyes (such as tartrazine, which is preferably present at a trace amount concentration of between 0 wt % or about 1x10⁻⁵ wt % to about 0.1 wt %). Some of the other ingredients listed above are discussed in further detail below.

### Pigment

The pigment may be organic or inorganic. Preferably the pigment is inorganic.

When organic, the pigment may be carbon black, an azo compound, xanthene, quinone, FD&C pigment, D&C pigment, or a lake pigment.

A FD&C pigment is known in the art and approved by the Federal Food, Drug and Cosmetic Act (FD&C Act).

A D&C pigment is known in the art and approved by US Food and Drug Administration.

An azo compound is known in the art and relates to a compound having a divalent -N=N- between two carbon atoms.

A lake pigment is known in the art and may be manufactured by precipitating a dye with an inert binder, or mordant, usually a metallic salt. Examples of a lake pigment include aluminium lakes, strontium lakes, or barium lakes.

Examples of FD&C and D&C pigments include Red 6, Red 7, Red 30, Red 34, Yellow 5, Blue 1, or derivatives thereof.

The pigment may be surface treated, often with alkyl silanes, to improve dispersion in a hydrophobic media. The most common pigment surface treatment is triethoxycaprylylsilane.

When inorganic, the pigment may be iron oxide, especially red, yellow and black iron oxides, titanium dioxide, zinc oxide, potassium ferricyanide (K₃3Fe(CN)₆, potassium ferrocyanide K₄Fe(CN)₆.3H₂O, or potassium ferrocyanide dehydrate, or an oxide of, zinc, zirconium and/or cerium.

In one embodiment the pigment may be black iron oxide, red iron oxide, yellow iron oxide and/or titanium dioxide.

In one embodiment the composition comprises, one or more pigments at a concentration of between 0 wt % or about 0.1 wt % and about 20 wt %. Preferably the composition comprises one or more pigments at a concentration of between about 2.5 wt % and about 18 wt %. More preferably the composition comprises one or more pigments at a concentration of between about 5 wt % and about 15 wt %.

### Antioxidant

The antioxidant may be a polyphenolic agent. The antioxidant may comprise extracts from plants chosen from Raspberry, Oregano (e.g. *Origanum vulgare*), Green tea (for example green leaves of *Camellia sinensis*), White tea (for example *Camellia sinensis*), Blueberry extract (for example *Vaccinium cyanococcus*), French maritime pine bark (for example *Pinus pinaster,* sold under the trade name Pycnogenol), Rosemary (for example *Rosmarinus officialis*), Grape, including grape seed (for example *Vitis vinifera*), Fennel (for example *Foeniculi fructus*), *Caragana sinica,* Marjoram (for example *Origanum majorana*), Crocus (for example *Crocus sativus*), Apple (for example *Malus domestica*), Mulberry (for example *Morus alba*), Ginseng (for example *Panax ginseng*), Coffee, Green coffee, Cherry (for example *Prunus avium*), Snow algae (for example *Chlamydomonas nivalis*), Emblica (for example *Phyllanthus emblica*), Gingko (for example *Gingko biloba*), Moringa (for example *Moringa oleilera*), Ginger (for example *Zingiberaceae*), Magnolia (for example *Magnolioideae virginiana*), French saffron, Edelweiss (for example *Leontopodium alpinium*), White lotus (for example *Nymphaea alba*), Turmeric root, Marshmallow (for example *Althaea officianlis*), Burdock (for example *Arctium lappa*), Bilberry (for example *Vaccinium myrtillus*), Cranberry (for example *Vaccinium oxycoccus*), Pomegranate nectar (for example *Punica granatum*), Sage (for example *Salvia officinalis*), Thyme (for example *Thymus vulgaris*), Sunflower (for example *Helianthus annuus*), wild carrot (for example *Daucus carota*), Hop (for example *Humulus lupulus*), Witch Hazel (for example *Hamamelis*), Oak (for example *Quercus*), Camellia (for example *Theacea*), Red clover (for example *Tritolium pratense*), Flax (for example *Linium usitatissimum*), lemon (for example *Citrus limon*), birch (for example *Betula*), cornflower, (for example *Centaurea cyanus*), geranium, polygonum, soy (for example *Glycine max*), and mixtures thereof.

In one embodiment the antioxidant polyphenolic agent may be an extract from a plant chosen from mulberry, ginseng, grape, oregano, grape, sage, sunflower, maritime pine bark, rosemary, marjoram, crocus, french saffron, wild carrot, hop, coffee, green coffee, witch hazel, oak, camellia, red clover, flax, ginger, magnolia, edelweiss, burdock and mixtures thereof.

Active polyphenolic species sourced from the above list of plants include those chosen from apigenin, luteolin, quercetin, kaempferol, naringenin, hesperetin, catechin, gallocatechin, cyaniding, pelargonidin, daidzein, caffeic acid, chlorogenic acid, romsmarinic acid, gallic acid, resveratrol, ferulic acid, epigallocatechin gallate, piceatannol, secoisolariciresinol, isotaxiresinol, Miyabenol c, Luteolin and mixtures thereof.

The amounts of antioxidant plant polyphenolic agents used in the present invention are expressed as dry weights of the extract, as understood by a man skilled in the art. In one embodiment the composition comprises one or more antioxidants (plant extracts) at a concentration of between 0 wt % or about 0.005 wt. % and about 10 wt. %. Preferably the antioxidant (plant extract) is present at a concentration of between about 0.01 wt. % and about 7 wt. %. More preferably the antioxidant (plant extract) is present at a concentration of between about 0.01 wt. % and about 5 wt. %.

### Skin Conditioning Agent

The composition of the present invention may optionally comprise a skin conditioning agent. The skin conditioning agents may be chosen from humectants, emollients, moisturisers, or mixtures thereof.

The skin conditioning agents may be chosen from guanidine, urea, glycolic acid, glycolate salts, salicylic acid, lactic acid, lactate salts, aloe vera, polyhydroxy alcohols (such as sorbitol, mannitol, xylitol, erythritol, glycerol, hexanetriol, butanitriol, (di) propylene glycol, butylene glycol, hexylene glycol, polyethylene glycol), sugars (for example fructose, glucose, xylose, honey, mannose, xylose), gluconodeltalactone, starches and derivatives thereof, pyrrolidone, carboxylic acid, hyaluronic acid and salts thereof (such as sodium hyaluronate), lactamide monoethanolamine, acetamide monoethanolamine, panthenol, allantoin, glycerine, ethylhexyl glycerine, arabinoglactan, PPG-15 stearyl ether, ethylhexyl stearate, cetyl dimethicone, octyldodecanol, PPG-20 methyl glucose ether, isopropyl myristate, isopropyl palmitate, isopropyl laurate, isodecyl laurate, isodecyl neopentanoate, isohexadecane, pentaerythrityl tetraisostearate, caprylic/capric triglyceride, canola oil, sunflower oil (*Helianthus annus*), olive oil (*Olea europea*), cottonseed oil (*Gossypium herbaceum*), jojoba oil (*Simmondsia chinensis*), shea butter (*Butyrospermum parkii*), cocoa butter (*Theobroma cacao*), cupuacu butter (*Theobroma grandiflorum*), avocado oil (*Persea gratissima*), liquid paraffin, dimethicone, phenyl trimethicone, cyclopentasiloxane, dimethiconol, , bisaccharide gum, isononyl isononoate, carnauba wax and/or petrolatum.

In one embodiment, the composition comprises one or more skin conditioning agents at a concentration of between 0wt% or 0.01wt% and 20 wt %. Preferably the skin conditioning agent is present at a concentration of between 0.1 wt % and 10 wt %. More preferably the skin conditioning agent is present at a concentration of between 0.5 wt % and 7wt%.

### Salicylic Acid Compound

The compositions of the present invention may optionally comprise a salicylic acid compound, its esters (such as alkylated salicylate, for example octyl salicylate), its salts, or combinations thereof. In one embodiment the salicylic acid compound is salicylic acid or octyl salicylic acid.

In one embodiment the composition comprises one or more salicylic acid compounds as described above at a concentration of between 0 wt % or about 0.0001 wt % and about 25 wt %. Preferably the salicylic acid compound is present at a concentration of between about 0.001 wt % to about 15 wt %. More preferably the salicylic acid compound is present at a concentration of between about 0.01 wt % to about 10 wt %. More preferably the salicylic acid compound is present at a concentration of between about 0.1 wt % to about 5 wt %. More preferably the salicylic acid compound is present at a concentration of between about 0.2 wt % to about 2 wt %.

### Emulsifier

The compositions of the present invention may optionally comprise an emulsifier. The emulsifier helps disperse and suspend an aqueous water phase within an oil phase or *vice versa.* Suitable emulsifiers include all those suitable for the purpose and known by those skilled in the art for use in skin care products. Preferably these emulsifiers have an HLB value of or less than 14, more preferably from 2 to 14, and still more preferably from 4 to 14.

A wide variety of silicone emulsifiers are useful herein. These silicone emulsifiers are typically organically modified organopolysiloxanes, also known to those skilled in the art as silicone surfactants. Useful silicone emulsifiers include dimethicone copolyols. These materials are polydimethyl siloxanes which have been modified to include polyether side chains such as polyethylene oxide chains, polypropylene oxide chains, mixtures of these chains, and chains comprising moieties derived from both ethylene oxide and propylene oxide. Other examples include alkyl-modified dimethicone copolyols, in other words compounds which comprise C2-C30 pendant side chains. Still other useful dimethicone copolyols include materials having various cationic, anionic, amphoteric and zwitterionic pendant moieties.

Emulsifiers also include various non-ionic and anionic emulsifying agents such as sugar esters and polyesters, alkoxylated sugar esters and polyesters, C1-C30 fatty acid esters of C1-C30 fatty alcohols, alkoxylated derivatives of C1-C30 fatty acid esters of C1-C30 fatty alcohols, alkoxylated ethers of C1-C30 fatty alcohols, polyglyceryl esters of C1-C30 fatty acids, C1-C30 esters ofpolyols, C1-C30 ethers ofpolyols, alkyl phosphates, polyoxyalkylene fatty ether phosphates, fatty acid amides, acyl lactylates, soaps, and mixtures thereof. Nonlimiting preferred examples of these non-silicon-comprising emulsifiers include: polyethylene glycol 20 sorbitan monolaurate (Polysorbate 20), polyethylene glycol 5 soya sterol, Steareth-20, Ceteareth-20, PPG-2 methyl glucose ether distearate, Ceteth-10, Polysorbate 80, cetyl phosphate, potassium cetyl phosphate, diethanolamine cetyl phosphate, Polysorbate 60, glyceryl stearate, PEG-100 stearate, polyoxyethylene 20 sorbitan trioleate (Polysorbate 85), sorbitan monolaurate, polyoxyethylene 4 lauryl ether sodium stearate, polyglyceryl-4 isostearate, hexyl laurate, steareth-20, ceteareth-20, PPG-2 methyl glucose ether distearate, ceteth-10, diethanolamine cetyl phosphate, glyceryl stearate, PEG-100 stearate, and mixtures thereof.

### Surfactants

The composition of the present invention may further comprise one or more surfactants, including but not limited to, anionic surfactants (for example sodium lauryl sulphate, sodium laureth sulphate, ammonium laureth sulphate, disodium laureth sulfosuccinate and sodium C12-15 pareth-12 carboxylate), amphoteric/zwitterionic surfactants (for example cocamidopropyl betaine, sodium cocoamphoacetate and cocamidopropyl hydroxysultaine), non-ionic surfactants (for example cocamide DEA, cocamide MEA, decyl glucoside, lauryl glucoside), and cationic surfactants (for example cetrimonium chloride, behentrimonium chloride and benzalkonium chloride).

In embodiments where one or more surfactants are present in the cosmetic composition, the one or more surfactants may be present at a concentration between about 0.1 wt % and about 10 wt %. Preferably the one or more surfactants are present at a concentration between about 0.25 wt % and about 7.5 wt %. More preferably the one or more surfactants are present at a concentration between about 0.5 wt % and about 6 wt %. More preferably the one or more surfactants are present at a concentration between about 0.5 wt % and about 5 wt %.

### Preservatives

Preservatives may be added to the composition such as benzoic acid, sodium benzoate, sorbic acid, potassium sorbate, 2-bromo2-nitropropane-1,3-diol (bronopol, which is available commercially under the trade name Myacide ®), benzyl alcohol, benzoic acid, sodium benzoate, diazolidinyl urea, imidazolidinyl urea, methyl paraben, phenoxyethanol, ethyl paraben, propyl paraben, sodium methyl paraben, sodium dehydroacetate, dehydroacetic acid, polyhexamethylenebiguanide hydrochloride, isothiazolone, chlorhexidine digluconate, chlorphensin and/or sodium propyl paraben. In one embodiment, the cosmetic composition of the invention does not comprise parabens.

In one embodiment the composition comprises one or more preservatives at a concentration of between 0 wt % or 0.001 wt % and about 5 wt %. Preferably the composition comprises one or more preservatives at a concentration of between about 0.01 wt % and about 4 wt %. More preferably the composition comprises one or more preservatives at a concentration of between about 0.1 wt % and about 2.5 wt %.

### Sequestering agents or chelating agents.

Sequestering agents or chelating agents may be added to the composition, such as ethylenediamine tetraacetic acid (EDTA) and salts thereof (for example dipotassium EDTA, disodium EDTA or tetrasodium EDTA), sodium phytate, trisodium ethylene diamine disuccinate, and/or tetrasodium glutamate diacetate.

In one embodiment the composition comprises one or more sequestering agents or chelating agents at a concentration of between 0 wt % or about 0.001 wt % and about 10 wt %. Preferably the composition comprises one or more sequestering agents or chelating agents at a concentration of between about 0.01 wt % and about 8 wt %. More preferably the composition comprises one or more sequestering agents or chelating agents at a concentration of between about 0.1 wt % and about 5 wt %.

### Vitamins

The cosmetic composition of the invention may further comprise one or more vitamins. For example, the cosmetic composition may further comprise vitamin B, vitamin B1 to vitamin B12, vitamin C, vitamin D, vitamin E, vitamin K, vitamin H, derivatives thereof, provitamins thereof (such as pro-vitamin B5 (panthenol)), or combinations thereof. In one embodiment where one or more vitamins are present, the vitamin is vitamin C and/or vitamin E.

In one embodiment the one or more vitamins are present at a concentration of between about 0.0001 wt % and about 50 wt %. Preferably the one or more vitamins are present at a concentration of between about 0.001 wt % and about 10 wt %. More preferably the one or more vitamins are present at a concentration of between about 0.01 wt % and about 8 wt %. More preferably the one or more vitamins are present at a concentration of between about 0.1 wt % and about 5 wt %.

### Waxes

The composition may include waxes such as cetearyl alcohol, Candelilla wax, Carnauba wax, lanolin wax, microcrystalline wax, ozokerite wax, paraffin wax, polyethylene wax or beeswax, cocoa butter

In one embodiment the composition comprises one or more waxes at a concentration of between 0 wt % or 0.01 wt % and about 10 wt %.

### Polar Solvent

The polar solvent is a cosmetically acceptable medium or a skin care acceptable medium. The polar solvent may include water, or an alcohol having 1 to 4 (or 2 to 3) carbon atoms. Examples of suitable alcohols include ethanol, propanol or iso-propanol.

In one embodiment the polar solvent is water; and in another embodiment the polar solvent is ethanol.

### Perfumes

Perfumes may be added to the composition of the present invention at a concentration of between 0 wt % or about 0.01% and 2 wt %.

### pH adjusting agents

The composition may also include pH adjusting agents such as potassium hydroxide, sodium hydroxide, amino methyl propanol sodium citrate and/or triethanolamine. The composition may be buffered by means well known in the art, for example by use of buffer systems comprising succinic acid, citric acid, lactic acid, and acceptable salts thereof, phosphoric acid, mono-or disodium phosphate and sodium carbonate. Suitably, the composition may have a pH of between about 3 and about 10, preferably between about 4 and about 8, more preferably between about 5 and about 7.

In one embodiment, the composition comprises one or more pH adjusting agents at a concentration of between 0 wt % or about 0.01 wt % and 10 wt %.

### Thickener, viscosity modifying agent or gelling agent

A thickener, viscosity modifying agent and/or gelling agent may be added to the composition. Preferably the thickener, viscosity modifying agent or gelling agent is polymeric. Examples of such polymeric thickeners, viscosity modifying agents and/or gelling agents include acrylic acid polymers, for example available commercially under the trade name Carbopol® or Ultrez® (both Lubrizol), taurate copolymers such as acryloyl methyl taurate-vinylpyrrolidone copolymers, alkylated polyvinylpyrrolidone copolymers (such as Anatron™V220), hydroxyethylacrylate/sodium acryloyldimethyl taurate copolymers, modified celluloses, for example hydroxyethylcelluloses available commercially under the trade name Natrosol® (Hercules), hydroxypropylmethyl celluloses, block polymers of ethylene oxide and propylene oxide (for example, those available from BASF Wyandotte under the trade name "Pluronic"®), decadiene crosspolymers (available under the trade name Stabilez® 60), Aristoflex®AVC (Clariant), xanthan gums, starches, or modified starches (such as a metal salt of starches, for example aluminium salts of the reaction product of 1-octenylsuccinic anhydride with starches), sodium polyacrylates, polyvinyl alcohols, and alkyl galactmanans available under the trade name N-Hance® from Hercules.

Alternatively the thickener, viscosity modifying agent or gelling agent may be non-polymeric. Examples of such non-polymeric thickener, viscosity modifying agent and gelling agents include amine oxides, ethoxylated fatty alcohols, salts (such magnesium chloride, sodium chloride) phthalic acid amides and fatty alcohols. In addition, the non-polymeric thickener may be inorganic. Inorganic thickeners include silica and clay materials such as bentonite, hectorite and montmorillonite. Specific examples of clay materials include disteardimonium hectorite and stearalkonium hectorite (these materials form part of the Bentone® gel range from Elementis). These inorganic thickeners may be hydrophobically modified. Examples of silica that have been hydrophobically modified are silica silylate and silica dimethyl silylate (available as part of the Aerosil® range from Evonik).

### Sunscreen Agent

The cosmetic composition of the invention may further comprise one or more sunscreen agents, including but not limited to inorganic sunscreen agents (for example microfine titanium dioxide and/or microfine zinc oxide) and organic sunscreen agents (for example p-aminobenzoic acids, esters and derivatives thereof, such as 2-ethylhexyl p-dimethyl-aminobenzoate), methoxycinnamate esters (for example 2-ethylhexyl p-methoxycinnamate, 2-ethoxyethyl p-methoxycinnamate or α,β-di-(p-methoxycinnamoyl)-α'-(2ethylhexanoyl)-glycerin), benzophenones (for example oxybenzone), dibenzoylmethanes (for example 4-(tert-butyl)-4'-methoxydibenzoylmethane), 2-phenylbenzimidazole-5 sulfonic acid and salts thereof, alkyl-β,β-diphenylacrylates (for example alkyl α-cyano-β,β-diphenylacrylates such as octocrylene) triazines (such as 2,4,6-trianilino-(p-carbo-2-ethyl-hexyl-1-oxy)-1,3,5 triazine) and/or camphor derivatives (such as methylbenzylidene camphor). In one embodiment the composition comprises one or more sunscreen agents at a concentration of between about 0.01 wt % to about 10 wt %.

### Formulation

The compositions of the present invention may be in the form of an emulsion, a powder, a spray (with or without a propellant), an aqueous solution (such as for wipes), a serum, a balm, or a gel. Preferably the composition is an emulsion. Some of the formulations listed above as discussed in further detail below.

### Emulsion

The emulsion may be a water-in-silicone, a water-in-oil, or an oil-in-water composition. In one embodiment the emulsion is a water-in-silicone composition.

When the emulsion is in the form of a water-in-silicone emulsion or water-in-oil emulsion, the aqueous phase may be present at a concentration of between about 15 wt % and about 55 wt %. Preferably the aqueous phase is present at a concentration of between about 25 wt % and about 45 wt %.

When the emulsion is in the form of a water-in-oil emulsion or oil-in-water emulsion, the emulsion may comprise an organic oil. The organic oil may be volatile or non-volatile. The organic oil may include a diluent, a solvent, a polyolefin polymer, an ester oil or combination thereof.

The term "ester oil" means an oil that is liquid at room temperature (25 °C) comprising at least one ester functional group. The ester oil used herein is chosen, for example, from monoesters.

The ester oil may, for example, be chosen from the monoesters of formula R¹COOR² wherein R¹ may be selected from linear and branched hydrocarbon-based chains comprising from 4 to 30, or 6 to 24, or 7 to 20 carbon atoms, and R² may be chosen from branched hydrocarbon-based chains comprising from 3 to 40 carbon atoms, such as from 10 to 30 carbon atoms and further such as from 16 to 26 carbon atoms.

Examples of the ester oils that may be mentioned include isodecyl neopentanoate; isocetyl octanoate; isononyl isononanoate, isodecyl isononanoate, tridecyl isononanoate; hexyl laurate, 2-hexyldecyl laurate; isopropyl myristate, isocetyl myristate, isotridecyl myristate, 2-octyldodecyl myristate; isopropyl palmitate, 2-ethylhexyl palmitate, isooctyl palmitate, isocetyl palmitate, isodecyl palmitate, isostearyl palmitate, 2-octyldecyl palmitate; isopropyl isostearate, 2-octyldodecyl stearate, isostearyl isostearate, and 2-octyldodecyl erucate.

### Gel

When in the form of a gel, the composition of the present invention may comprise one or more thickeners, viscosity modifying agents and/or a gelling agents at a concentration of between about 0.1 wt % and about 10 wt %. Preferably the composition comprises one or more thickeners at a concentration of between about 0.5 wt % and about 5 wt %. More preferably the composition comprises one or more thickeners at a concentration of between about 0.5 wt % and about 3 wt %. The thickener may be a polymeric thickener.

### Methods and Uses

As described in the Example, the compositions of the present invention provide the following improved benefits when compared to similar prior art compositions: ease of application, flawless finish upon application, evened out skin tone upon application, a less cakey look upon application, a more youthful look upon application, less noticeable pores after application and less noticeable fine lines after application.

Thus, in a further aspect, the present invention provides a method of imparting a benefit to a composition of at least one of easy application, flawless finish upon application, evened out skin tone upon application, a less cakey look upon application, a more youthful look upon application, less noticeable pores after application and less noticeable fine lines after application, comprising application of the composition of the present invention topically to the skin. In one embodiment the method imparts a benefit that relates to anti-ageing, in other words the method imparts a benefit of at least one of evened out skin tone upon application, a more youthful look upon application, less noticeable pores after application and less noticeable fine lines after application. In an alternative embodiment, the method imparts the benefit of easy application.

In a further aspect, the present invention provides a cosmetic use of the composition of the present invention. In one embodiment, the cosmetic use imparts a benefit of at least one of easy application, flawless finish upon application, evened out skin tone upon application, a less cakey look upon application, a more youthful look upon application, less noticeable pores after application and less noticeable fine lines after application. In one embodiment the use imparts a benefit that relates to anti-ageing, in other words the use imparts a benefit of at least one of evened out skin tone upon application, a more youthful look upon application, less noticeable pores after application and less noticeable fine lines after application. In an alternative embodiment, the use imparts the benefit of easy application.

The uses and methods described above are known to the skilled person as not encompassing therapeutic or medical treatment. In other words the disclosed uses and methods relate to non-therapeutic uses and methods.

### EXAMPLE

### Foundation Study

### Material and Methods:

An example foundation composition of the present invention was tested comprising 3 wt % of platelets in the form of mica treated with triethoxycaprylylsilane, 3 wt % of particles in the form of nylon 6/12 powder, 4 wt % of Dow Corning RSN-0749 Resin (which includes 2 wt % of film-forming polymer in the form of trimethylsiloxysilicate), 4 wt % of elastomer in the form of a dimethicone and dimethicone crosspolymer (Dow Corning EL-9240), caprylyl methicone, preserving agents, cyclopentasiloxane, disteardimonium hectorite, propylene carbonate, retinyl palmitate, ethylhexyl methoxycinnamate, cyclohexasiloxane, cyclopentasiloxane, tocopheryl acetate, PEG-9 dimethicone, bis-isobutyl PEG/PPG-10/7dimethicone copolymer, PEG/PPG dimethylallylether, pigments treated with triethoxycaprylsilane, titanium dioxide nanoparticles, silica, dimethicone, ascorbyl glucoside, potassium hydroxide, tetrasodium EDTA, magnesium sulfate, butylene glycol, denatured alcohol, glycerine and purified water (Foundation M). This foundation was compared against a gold standard, more specifically a popular anti-ageing foundation on the market comprising a "Light Optimising Complex" (specifically a combination of silica microspheres and platelets in the form of talc and mica). The gold standard foundation comprises aqua, cyclopentasiloxane, isononyl isononanoate, butylene glycol, talc, dimethicone, polyglyceryl-3 ricinoleate, sorbitan oleate, PEG-30 dipolyhydroxystearate, magnesium sulfate, silica, phenoxyethanol, magnesium stearate, polyhydroxystearic acid, dimethiconol, disteardimonium hectorite, dimethicone crosspolymer, parfum, sorbic acid, *Avena sativa* kernel extract, dimethicone/vinyl dimethicone crosspolymer, hydroxyethyl urea, lauroyl lysine, *Camellia sinensis* leaf extract, algin, sodium benzoate, *Acacia senegal* gum, *Lapsana communis* extract, *Plantago major* seed extract, α-isomethyl ionone, linalool, hexyl cinnamal and L-limonene. The person skilled in the art knows that for a composition having an INCI list that ingredients featured higher in a list are present at higher concentrations than ingredients listed thereafter.

Foundation M and the gold standard foundation were tested by 35 volunteers. Volunteers were asked to apply these foundations as usual and then fill in a questionnaire.

### Results:

The volunteer results are shown in the Table 1 below:

| Claim | % Positive Result | |
|---|---|---|
| | Foundation M | Gold Standard Foundation |
| Immediately after application this foundation ... | | |
| it was easy to apply | 98 | 85 |

| This foundation gave ... | | |
|---|---|---|
| a flawless finish to my skin | 87 | 83 |
| an evened out skin tone | 89 | 85 |
| did not leave my skin looking cakey | 83 | 78 |
| a more youthful look | 81 | 61 |

| Whilst wearing foundation ... | | |
|---|---|---|
| my pores were less noticeable | 85 | 74 |
| my fine lines were less noticeable | 85 | 65 |

The results indicate that a composition comprising a concentration of platelets that is similar to the concentration of particles clearly reduces the signs of ageing upon application when compared to an equivalent gold standard foundation that has a concentration of platelets that is dissimilar to the concentration of particles.

Despite the presence of a dimethicone and dimethicone crosspolymer in both the gold standard foundation and Foundation M, it is noted that Foundation M was easier to apply compared to the gold standard foundation. This suggests that, in compositions comprising a similar concentration of particles and platelets such as Foundation M, the presence of elastomer provides a surprisingly improved ease of application compared to formulations such as the gold standard foundation where the concentration of the particles and platelets is dissimilar.

## Claims

1. A composition comprising particles and platelets, wherein the particles and platelets are present at a concentration (w/w) ratio of between about 30:70 and about 70:30.

2. The composition of claim 1, wherein the composition is a cosmetic composition.

3. The composition of claim 2, wherein the composition is a colour cosmetic.

4. The composition of claim 3, wherein the composition is a pigmented emulsion.

5. The composition of any one of claims 1 to 4, wherein the concentration (w/w) ratio of particles to platelets is between about 40:60 and about 60:40, preferably between about 45:55 and 55:45, more preferably about 50:50.

6. The composition of any one of claims 1 to 5, wherein the particles and/or the platelets are present at a concentration of between about 1 wt % and about 5 wt %, preferably between about 2 wt % and about 4 wt %, more preferably between about 2.5 wt % and about 3.5 wt %, more preferably about 3 wt %.

7. The composition of any one of claims 1 to 6, wherein the particles are polymer particles.

8. The composition of claim 7, wherein the particles are nylon particles.

9. The composition of any one of claims 1 to 8, wherein the platelets are derived from a mineral.

10. The composition of claim 9, wherein the platelets are talc platelets or mica platelets.

11. The composition of any one of claims 1 to 10, wherein the composition further comprises an elastomer.

12. The composition of claim 11, wherein the elastomer is present at a concentration of between about 0.5 wt % and about 10 wt %, preferably between about 1 wt % and about 8 wt %, more preferably between about 2 wt % and about 6 wt %, more preferably between about 3 wt % and about 5 wt %, more preferably about 4 wt %.

13. The composition of claim 11 or claim 12, wherein the elastomer is a non-emulsifying elastomer, preferably a dimethicone crosspolymer.

14. The composition of any one of claims 1 to 13, wherein the composition further comprises a film-forming polymer.

15. The composition of claim 14, wherein the film-forming polymer is present at a concentration of between about 0.1 wt % and about 10 wt %, preferably between about 0.5 wt % and about 6 wt %, more preferably between about 1 wt % and about 3 wt %, more preferably about 2 wt %.

16. The composition of claim 14 or claim 15, wherein the film-forming polymer comprises silicon, preferably the film-forming polymer is a siloxysilicate, more preferably trimethylsiloxysilicate.

17. A method of imparting a benefit to a composition of at least one of easy application, flawless finish upon application, evened out skin tone upon application, a less cakey look upon application, a more youthful look upon application, less noticeable pores after application and less noticeable fine lines after application, comprising application of the composition of any one of claims 1 to 16 topically to the skin.

18. The cosmetic use of the composition of any one of claims 1 to 16.

19. The cosmetic use of claim 18, wherein the cosmetic use imparts a benefit of at least one of easy application, flawless finish upon application, evened out skin tone upon application, a less cakey look upon application, a more youthful look upon application, less noticeable pores after application and less noticeable fine lines after application.
